# EUROPÄISCHE PATENTSCHRIFT

(11) **EP 1 585 564 B1**
(45) Veröffentlichungstag und Bekanntmachung des Hinweises auf die Patenterteilung: **12.06.2013**
(21) Anmeldenummer: 04701912.0
(22) Anmeldetag: 14.01.2004
(51) Int. Cl.: A61M 1/16

(54) **VERFAHREN UND VORRICHTUNG ZUR VERSORGUNG EINER DIALYSEVORRICHTUNG MIT DIALYSIERFLÜSSIGKEIT**
METHOD AND DEVICE FOR SUPPLY OF A DIALYSIS UNIT WITH DIALYSIS FLUID
PROCEDE ET DISPOSITIF POUR ALIMENTER UN APPAREIL DE DIALYSE EN LIQUIDE DE DIALYSE

(30) Priorität: 24.01.2003 DE 10302691
(43) Veröffentlichungstag der Anmeldung: 19.10.2005
(73) Patentinhaber: Fresenius Medical Care Deutschland GmbH, 61352 Bad Homburg v.d.H. (DE)
(72) Erfinder: REMKES, Gerard, 63667 Eichelsdorf (DE); WIESEN, Gerhard, 61352 Bad Homburg v.d.H. (DE)
(74) Vertreter: Oppermann, Frank
(86) Internationale Anmeldenummer: PCT/EP2004/000188
(87) Internationale Veröffentlichungsnummer: WO 2004/064886

(56) Entgegenhaltungen:
- US-A- 4 153 554
- US-A- 5 744 027
- US-A1- 2001 040 127

## Beschreibung

Die Erfindung betrifft eine Vorrichtung zur Versorgung eines Dialysators einer Dialysevorrichtung mit Dialysierflüssigkeit.

Es ist heute allgemein üblich, zur Herstellung von Dialysierflüssigkeit für Hämodialysevorrichtungen vorgefertigte Dialysierflüssigkeitskonzentrate zu verwenden, die in den Dialysevorrichtungen mit Wasser verdünnt werden. In Dialysezentren werden Dialysierflüssigkeitskonzentrate entweder als vorgefertigtes Produkt in Kanistern oder Beuteln zur Verfügung gestellt oder über ein Ringleitungssytem aus einem zentralen Tank bereitgestellt.

Die Dialysierflüssigkeit wird dem Dialysator der Dialysevorrichtung zugeführt, der durch eine semipermeable Membran in eine Dialysierflüssigkeitskammer und eine Blutkammer unterteilt ist. Während durch die Blutkammer kontinuierlich Blut des Patienten strömt, fließt durch die Dialyiserflüssigkeitskammer im Gegenstrom fortlaufend Dialysierflüssigkeit.

Zentral zur Verfügung gestellte Dialysierflüssigkeitskonzentrate sind für den Anwender einfach zu handhaben, sie haben aber den Nachteil, dass die Dialysierflüssigkeit nicht individuell auf die Bedürfnisse des Patienten abgestimmt werden kann. Dezentral bereitgestellte Konzentrate erlauben zwar eine individuelle Anpassung der Dialysierflüssigkeit an den Patienten, sie müssen aber für jede Dialysebehandlung in Kanistern oder Beuteln an die Dialysevorrichtung gebracht werden. Üblicherweise ist ein Kanister mit 5 bis 6 Liter Säurekonzentrat sowie ein Beutel mit 650 bis 750g Natriumbicarbonat erforderlich.

Da die zentral bereitgestellten Konzentrate je nach Bedarf abgerufen werden können, ergeben sich keine Restmengen. Dem gegenüber werden die vorkonfektionierten Konzentrate, die nur für eine Behandlung bereitgestellt werden, in der Praxis nicht aufgebraucht. Eine korrekte Entsorgung der Verpackungsmaterialien durch Granulieren oder Verbrennen ist aber nur nach einer vollständigen Entleerung der Kanister oder Beutel möglich, so dass die nach der Behandlung in den Kanistern oder Beuteln befindlichen Restmengen verworfen werden müssen. Im Übrigen entsteht durch das Verwerfen überschüssiger Konzentratmengen ein materieller Schaden.

Zur Herstellung der Dialysierflüssigkeit aus Konzentraten und Wasser sind verschiedene Vorrichtungen bekannt. Die US-A-4,895,657 beispielsweise beschreibt eine Proportionierungsvorrichtung, bei der zwei Flüssigkonzentrate in Konzentratbehältern bereitgestellt und zur Verdünnung in einem vorgegebenen Volumenverhältnis mit Wasser vermischt werden. Normalerweise werden die Konzentrate als "35-fach" bezeichnet, d.h. einem Volumenteil Konzentrat wird soviel Wasser zugesetzt, dass das Gesamtvolumen 35 Volumenteile erreicht. Praktisch bedeutet dies, dass ein Volumenteil Konzentrat und 34 Volumenteile Wasser zusammengegeben werden.

Die US 5 744 027 A beschreibt eine Dialysevorrichtung, die über eine Einrichtung zur Versorgung der Maschine mit Dialysierflüssigkeit verfügt. Die Einrichtung zur Versorgung der Maschine mit Dialysierflüssigkeit weist einen Behälter zur Aufnahme von Dialysierflüssigkeitskonzentrat und eine Einheit zum Mischen des Dialysierflüssigkeitskonzentrats mit Wasser in einem vorgegebenen Volumenverhältnis zur Herstellung der Dialysierflüssigkeit auf. Darüber hinaus verfügt die Dialysevorrichtung über eine Steuer- und Recheneinheit, die derart ausgebildet ist, dass eine vorgegebene Dialysierflüssigkeitsrate einstellbar ist.

Der Erfindung liegt die Aufgabe zugrunde, eine Vorrichtung zur Versorgung eines Dialysators einer Dialysevorrichtung mit Dialysierflüssigkeit anzugeben, die eine bedarfsgerechte Bereitstellung von Dialysierflüssigkeit erlaubt. Diese Aufgabe wird erfindungsgemäß mit den Merkmalen des Patentanspruchs 1 gelöst.

Bei der erfindungsgemäßen Vorrichtung wird mindestens ein Dialysierflüssigkeitskonzentrat in mindestens einer Aufnahmeeinheit bereitgestellt. Als Aufnahmeeinheiten können beispielsweise Behälter, Beutel oder dgl. Verwendung finden.

Die erfindungsgemäße Vorrichtung beruht auf dem Grundprinzip, dass die Dialysierflüssigkeitsrate während der Dialysebehandlung derart eingestellt wird, dass zum Behandlungsende in der

Aufnahmeeinheit eine vorgegebene Restmenge an Dialysierflüssigkeitskonzentrat verbleibt oder keine Restmenge in der Aufnahmeeinheit verbleibt. Dabei erfolgt die Einstellung der Dialysierflüssigkeitsrate in Abhängigkeit von der vorgegebenen Menge an Dialysierflüssigkeitskonzentrat zu Beginn der Dialysebehandlung, dem vorgegebenen Volumenverhältnis zwischen Dialysierflüssigkeitskonzentrat und Wasser und der vorgegebenen Behandlungszeit. Die Dialysierflüssigkeitsrate kann während der Dialysebehandlung konstant sein oder auch variiert werden.

Die erfindungsgemäße Vorrichtung ermöglicht die Einstellung einer Dialysierflüssigkeitsrate, die über der für die Behandlung mindestens erforderlichen Rate liegt, so dass die Dialysance insgesamt erhöht wird.

Bei einer konventionellen Dialysebehandlung gibt der Arzt eine bestimmte Dialysedosis vor, indem er für einen bestimmten Typ eines Dialysators einen entsprechenden Blutfluss und Dialysierflüssigkeitsfluss sowie eine entsprechende Behandlungszeit einstellt. Die zur Verfügung gestellten Konzentratmengen sind in der Praxis dabei so bemessen, das sie für Behandlungen mit unterschiedlichen Dialysierflüssigkeitsflüssen im allgemeinen ausreichend sind. Daher wird das Konzentrat in der Praxis nicht vollständig verbraucht. Das erfindungsgemäße Verfahren bzw. die Vorrichtung macht davon Gebrauch, die verbleibende Konzentratmenge zur Einstellung einer höheren Dialysierflüssigkeitsrate zu nutzen. Von Vorteil ist, dass mit einer höheren Dialysierflüssigkeitsrate gleichzeitig die Dialysedosis erhöht wird, was für die Behandlung von Vorteil ist. Beispielsweise steigt die Effizienz eines Hohlfaserdialysators mit zunehmenden Dialysierflüssigkeitsflüssen bis zu einem asymptotischen Grenzwert.

Aus den obengenannten Gründen wird grundsätzlich angestrebt, dass die Dialysierflüssigkeit in der Aufnahmeeinheit zum Behandlungsende aufgebraucht ist. In der Praxis kann es aber in einzelnen Fällen sinnvoll sein, die Dialysierflüssigkeitsrate derart zu bemessen, dass als Reserve noch eine vorgegebene Restmenge an Dialyiserflüssigkeitskonzentrat in der Aufnahmeeinheit verbleibt. Diese Restmenge ist zum Beispiel dann erforderlich, wenn die Behandlung auf Grund von Komplikationen einmal oder mehrmals unterbrochen wird, wobei während der Unterbrechung der Behandlung die Dialysierflüssigkeit verworfen wird.

Mit der vorgegebenen Restmenge an Konzentrat kann dann die Dialysierflüssigkeit hergestellt werden, die für eine entsprechende Verlängerung der Behandlung notwendig ist, so dass die effektive Behandlungszeit wieder erreicht wird.

Die vorgegebene Restmenge an Dialysierflüssigkeit wird nach Ablauf der Behandlungszeit vorzugsweise abgeführt, so dass die Aufnahmeeinheit zur Entsorgung vollständig entleert ist. Vorzugsweise wird die vorgegebene Restmenge an Dialysierflüssigkeit in einem vorgegebenen Volumenverhältnis mit Wasser verdünnt, so dass das verdünnte Konzentrat ohne Probleme in einen Ablauf abgeführt werden kann.

Die vorgegebene Menge an Dialyiserflüssigkeitskonzentrat, das vorgegebene Volumenverhältnis und die vorgegebene Behandlungszeit können manuell eingegeben oder auch automatisch eingelesen werden. Beispielsweise können die Daten in Form eines Strichcodes auf den Behältern oder Beuteln hinterlegt und mit einem entsprechenden Lesegerät eingelesen werden.

Bei einer bevorzugten Ausführungsform der erfindungsgemäßen Vorrichtung wird die Dialysierflüssigkeitsrate, die über die gesamte Behandlungszeit eingestellt wird, vor Beginn der Dialysebehandlung aus der vorgegebenen Menge an Dialyiserflüssigkeitskonzentrat zu Beginn der Behandlung, dem vorgegebenen Volumenverhältnis von Konzentrat und Wasser und der vorgegebenen Behandlungszeit bestimmt. Diese Dialysierflüssigkeitsrate kann dann über die gesamte Behandlungszeit eingestellt werden, so dass in der Aufnahmeeinheit die vorgegebene Restmenge an Konzentrat oder keine Restmenge verbleibt.

Eine alternative Ausführungsform sieht vor, zunächst für ein vorgegebenes Zeitintervall, das einen Großteil der gesamten Dialysebehandlung ausmachen sollte, eine vorgegebene Dialysierflüssigkeitsrate einzustellen, wobei nach Ablauf des vorgegebenen Zeitintervalls erst die Dialysierflüssigkeitsrate bestimmt wird, die über die verbleibende Behandlungszeit einzustellen ist, so dass zum Behandlungsende in der Aufnahmeeinheit die vorgegebene Restmenge an Dialysierflüssigkeitskonzentrat oder keine Restmenge verbleibt. Hierzu wird die Dialysierflüssigkeitsrate nach Ablauf des vorgegebenen Zeitintervalls aus der in der Aufnahmeeinheit noch befindlichen Menge an Dialysierflüssigkeitskonzentrat, dem vorgegebenen Volumenverhältnis und der verbleibenden Behandlungszeit bestimmt. Die in der Aufnahmeeinheit befindliche Menge an Dialysierflüssigkeitskonzentrat wird aus der vorgegebenen Konzentratmenge zu Beginn der Dialysebehandlung und der verbrauchten Menge an Dialysierflüssigkeitskonzentrat bestimmt. Wenn die Proportionierung mit einer Pumpe erfolgt, bei der ein gleichbleibender Zusammenhang zwischen der Zahl der Arbeitszyklen, d.h. Hübe, Umdrehungen oder dgl. und dem dabei geförderten Volumen besteht, kann die verbrauchte Menge an Dialyiserflüssigkeitskonzentrat unabhängig von äußeren Betriebsbedingungen auf einfache Weise genau bestimmt werden. Für den Fall einer oder mehrerer Unterbrechungen der Dialysebehandlung während des vorgegebenen Zeitintervalls kann die Behandlungszeit entsprechend verlängert und auf der Grundlage der verlängerten Behandlungszeit dann die optimale Dialysierflüssigkeitsrate bestimmt werden.

Wenn mehrere Konzentrate in mehreren Aufnahmeeinheiten verwendet werden, kann eine Restmenge nur für eine der Aufnahmeeinheiten vorgegeben werden, wenn die Aufnahmeeinheiten ein unterschiedliches Fassungsvermögen haben und das Volumenverhältnis von Konzentrat und Wasser unterschiedlich ist. Die Berechnung der Dialysierflüssigkeitsrate sollte dann auf der Grundlage der vorgegebenen Konzentratmenge der Aufnahmeeinheit erfolgen, deren Konzentrat zuerst verbraucht ist.

Verschiedene Dialysevorrichtungen sehen einen Test vor Beginn der Dialysebehandlung in einem vorgegebenen Zeitintervall vor. Um die vorgegebene Menge an Dialysierflüssigkeitskonzentrat zu Beginn der Dialysebehandlung zu bestimmen, wird aus der vor dem Test in der Aufnahmeeinheit befindlichen Menge an Dialyiserflüssigkeitskonzentrat und der während des vorgegebenen Zeitintervalls verbrauchten Menge an Dialysierflüssigkeitskonzentrat die in der Aufnahmeeinheit vor Beginn der Dialysebehandlung befindliche Konzentratmenge bestimmt.

Im Folgenden werden zwei Ausführungsbeispiele der erfindungsgemäßen Vorrichtung unter Bezugnahme auf die Zeichnungen näher erläutert.

Es zeigen:

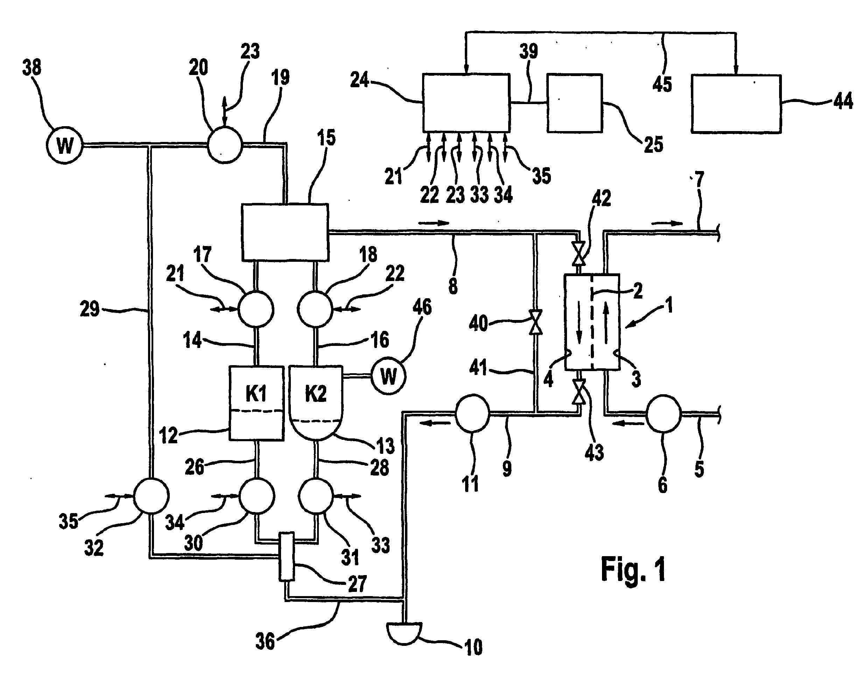

- Fig. 1: eine Vorrichtung zur Versorgung eines Dialysators einer Dialysevorrichtung zusammen mit der Dialysevorrichtung in stark vereinfachter schematischer Darstellung,
- Fig. 2a und 2b: die Konzentratmenge bzw. den Dialysierflüssigkeitsfluss als Funktion der Zeit für ein erstes Ausführungsbeispiel und
- Fig. 3a und 3b: Konzentratmenge bzw. Dialysierflüssigkeitsfluss als Funktion der Zeit Für ein zweites Ausführungsbeispiel.

Die Hämodialysevorrichtung weist einen Dialysator 1 auf, der durch eine semipermeable Membran 2 in eine Blutkammer 3 und eine Dialysierflüssigkeitskammer 4 unterteilt ist. Der Einlass der Blutkammer 3 ist mit einem Ende einer Blutzuführleitung 5 verbunden, in die eine Blutpumpe 6 geschaltet ist, während der Auslass der Blutkammer mit einem Ende einer Blutabführleitung 7 verbunden ist. Zu dem Einlass der Dialysierflüssigkeitskammer 4 führt eine Dialysierflüssigkeitszuführleitung 8, und von dem Auslass der Dialysierflüssigkeitskammer geht eine Dialysierflüssigkeitsabführleitung 9 ab, die zu einem Abfluss 10 führt. In die Dialysierflüssigkeitsabführleitung 9 ist eine Dialysierflüssigkeitspumpe 11 geschaltet. Während der Dialysebehandlung strömt Blut des Patienten durch die Blutkammer 3 des Dialysators 1, während im Gegenstrom Dialysierflüssigkeit durch die Dialysierflüssigkeitskammer 4 fließt.

Die Vorrichtung zur Versorgung des Dialysators mit Dialysierflüssigkeit ist im Allgemeinen Bestandteil der Dialysevorrichtung. Die Versorgungsvorrichtung kann grundsätzlich aber auch eine separate Einheit bilden. Im Folgenden wird die Versorgungsvorrichtung im Einzelnen beschrieben.

Zur Herstellung der Dialysierflüssigkeit werden zwei Konzentrate K1, K2 mit Wasser W in einem vorgegebenen Volumenverhältnis gemischt. Beispiele für in der Praxis übliche Angaben sind Volumenverhältnis "35-fach" oder "45-fach", d.h. ein Volumenteil Konzentrat und 34 bzw. 44 Volumenteile Wasser werden zusammengegeben. Bei der Verwendung von pulverförmigen Trockenkonzentrat anstelle von Flüssigkonzentrat wird die Masse des Pulvers zunächst in eine entsprechende Menge an Flüssigkonzentrat umgerechnet, damit das Konzentrat mit Wasser in dem vorgegebenen Volumenverhältnis gemischt werden kann.

Die Versorgungsvorrichtung verfügt über zwei Aufnahmeeinheiten für die beiden Konzentrate, von denen die eine ein Kanister 12, der mit 5 bis 61 Säurekonzentrat befüllt ist, und die andere ein Beutel 13 ist, der mit 650 bis 750g Natriumbikarbonat befüllt ist.

Von dem Kanister 12 geht eine erste Konzentratleitung 14 ab, die zu einer Mischkammer 15 führt, und von dem Beutel 13 geht eine zweite Konzentratleitung 16 ab, die zu der Mischkammer 15 führt. In die erste und zweite Konzentratleitung 14, 16 sind jeweils eine Proportionierungspumpe 17, 18 geschaltet. Zu der Mischkammer 15 führt ferner eine Wasserleitung 19, die an einer Wasserquelle 38 angeschlossen ist. In die Wasserleitung 19 ist ebenfalls eine Proportionierungspumpe 20 geschaltet.

Die Proportionierungspumpen 17, 18 und 20 sind über Daten- und Steuerleitungen 21, 22 und 23 an eine zentrale Steuer- und Recheneinheit 24 angeschlossen, die für die Proportionierungspumpen bestimmte Förderraten vorgeben, so dass die Konzentrate und Wasser jeweils in einem vorgegebenen Volumenverhältnis zur Herstellung der Dialysierflüssigkeit in der Mischkammer 15 gemischt werden. An dem Beutel 13 mit dem Trockenkonzentrat ist ein Wasserzugang 46 um Zuführen einer vorgegebenen Menge an Wasser vorgesehen. Um aus dem Trockenkonzentrat ein mit Wasser in einem vorgegebenen Volumenverhältnis zu mischendes Flüssigkonzentrat zu gewinnen, wird das Pulver zunächst in Wasser gelöst, das aus dem Wasserzugang in den Beutel zufließt.

Darüber hinaus verfügt die Versorgungsvorrichtung über eine Eingabeeinheit 25, die über eine Datenleitung 39 mit der zentralen Steuer- und Recheneinheit 24 kommuniziert.

Von dem Kanister 12 geht eine Entleerungsleitung 26 ab, die zu einer zweiten Mischkammer 27 führt, während von dem Beutel 13 eine zweite Entleerungsleitung 28 abgeht, die zu der Mischkammer 27 führt. Von der Wasserquelle 38 geht eine Wasserleitung 29 ab, die ebenfalls zu der Mischkammer 27 führt. In die erste und zweite Entleerungsleitung 26, 28 und in die Wasserleitung 29 sind jeweils Pumpen 30, 31, 32 geschaltet, die über Steuerleitungen 33, 34, 35 mit der zentralen Steuer- und Recheneinheit 24 verbunden sind. Von der Mischkammer 27 geht eine Ablaufleitung 36 ab, die zu dem Abfluss 10 führt.

Für die Unterbrechung der Dialysebehandlung, beispielsweise beim Auftreten von Komplikationen oder für die Durchführung eines Tests, sind eine Bypassleitung 40, in die ein Bypassventil 41 geschaltet ist, und ein Absperrventil 42 stromauf und ein Absperrventil 43 stromab der Dialysierflüssigkeitskammer 4 des Dialysators 1 vorgesehen. Wenn die Dialysebehandlung unterbrochen ist, strömt die Dialysierflüssigkeit durch die Bypassleitung 40 in den Ablauf 10, wobei die Dialysierflüssigkeitskammer 4 von Dialysierflüssigkeit nicht durchflossen wird.

Die Hämodialysevorrichtung verfügt ebenfalls über eine zentrale Rechen- und Steuereinheit 44, die über eine Datenleitung 45 mit der Steuer- und Recheneinheit 24 der Versorgungsvorrichtung kommuniziert.

Nachfolgend wird die Funktionsweise der Versorgungsvorrichtung im Einzelnen beschrieben.

Bei einer ersten Ausführungsform steuert die Steuer- und Recheneinheit 24 der Versorgungsvorrichtung die Pumpen derart an, dass die folgenden Verfahrensschritte durchgeführt werden.

Vor der Dialysebehandlung wird die in dem Kanister 12 und dem Beutel 13 jeweils befindliche Menge M₁, M₂ an Flüssigkonzentrat in die Eingabeeinheit 25 eingegeben. In die Eingabeeinheit 25 wird ferner die effektive Behandlungszeit T_{B} sowie das Volumenverhältnis V₁, V₂ von Konzentrat K1 bzw. K2 und Wasser W eingegeben.

Zur Herstellung der Dialysierflüssigkeit, die dem Dialysator 1 zugeführt werden soll, gibt die Steuer- und Recheneinheit 24 die Förderraten der Proportionierungspumpen 17,18, 20 derart vor, dass in der Mischkammer die Konzentrate K1, K2 jeweils mit Wasser in dem vorgegebenen Volumenverhältnis gemischt werden.

Während einer initialen Test- und Vorbereitungsphase schließt die Steuer- und Recheneinheit 44 der Dialysevorrichtung die Absperrventile 42, 43 und öffnet das Bypassventil 41, so dass die Dialysierflüssigkeit durch die Bypassleitung 40 für ein vorgegebenes Zeitintervall Tₜₑₛₜ in den Ablauf 10 strömt. Die Dialysierflüssigkeitsrate beträgt beispielsweise Qdₜ. Nach Ablauf des vorgegebenen Zeitintervalls Tₜₑₛₜ beginnt dann die eigentliche Dialysebehandlung.

Die Steuer- und Recheneinheit 24 berechnet nun aus den vorgegebenen Förderraten der Proportionierungspumpen 17 und 18 die Menge an Konzentrat, die während des Zeitintervalls Tₜₑₛₜ verbraucht wurde. Aus der Differenz der vorgegebenen Konzentratmenge M₁, M₂ und der in dem Zeitintervall Tₜₑₛₜ verbrauchten Konzentratmenge berechnet die Steuer- und Recheneinheit 24 nun die zu Beginn der eigentlichen Dialysebehandlung in dem Behälter 12 bzw. Beutel 13 befindliche Konzentratmenge.

Bei der Dialysebehandlung soll angestrebt werden, dass nach Möglichkeit beide Aufnahmeeinheiten vollständig entleert werden. Dies kann in der Praxis aber nicht erreicht werden, wenn der Inhalt des Kanisters 12 und des Beutels 13 nicht exakt aufeinander abgestimmt sind. Daher gibt die Steuer- und Recheneinheit 24 die Aufnahmeeinheit vor, der vollständig entleert werden soll. Im vorliegenden Ausführungsbeispiel wird angenommen, dass der Kanister 12 vollständig entleert werden soll. Grundsätzlich kann auch angestrebt werden, dass in dem Kanister 12 eine vorgegebene Restmenge verbleiben soll. Diese Vorgaben sind aber nur beispielhaft. Die Steuer- und Recheneinheit 24 kann beispielsweise vorgeben, welche Aufnahmeeinheit vollständig entleert werden soll, wobei in der anderen Aufnahmeeinheit eine möglichst geringe Restmenge verbleiben soll.

Die Steuer- und Recheneinheit 24 berechnet nun aus der nach dem Test zu Beginn der Dialysebehandlung (Zeitpunkt t₁) in dem Kanister 12 befindlichen Konzentratmenge Mₜ₁, der vorgegebenen Behandlungszeit T_{B} sowie dem vorgegebenen Volumenverhältnis V₁ von Säurekonzentrat K1 und Wasser W die Dialysierflüssigkeitsrate Qd, bei der nach Ablauf der Behandlungszeit das Konzentrat aufgebraucht ist, so dass in dem Kanister keine Restmenge an Konzentrat mehr verbleibt. Diese Dialysierflüssigkeitsrate wird an die zentrale Steuer- und Recheneinheit 44 der Dialysevorrichtung über die Datenleitung 45 weitergegeben, die dann die entsprechende Förderrate der Dialysierflüssigkeitspumpe 11 einstellt

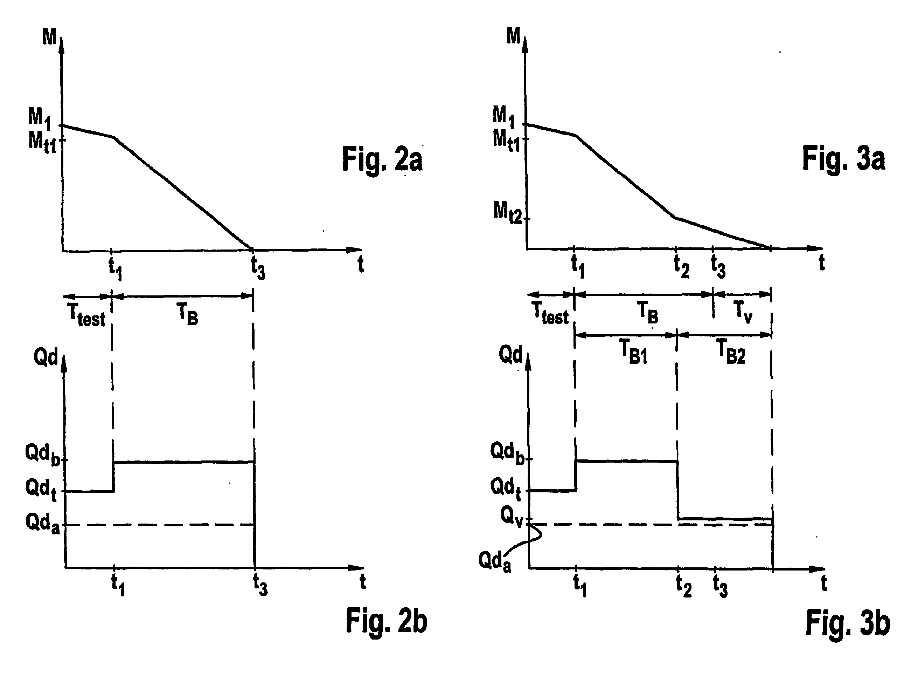

Die Figuren 2a und 2b zeigen die Konzentratmenge M in dem Kanister 12 bzw. die Dialysierflüssigkeitsrate Qd als Funktion der Zeit t. Deutlich ist zu erkennen, dass für den Test zum Zeitpunkt 0 eine erste konstante Dialysierflüssigkeitsrate Qdₜ eingestellt wird und für die eigentlichen Dialysebehandlung zum Zeitpunkt t₁ eine zweite konstante Dialysierflüssigkeitsrate Qd eingestellt wird, die derart bemessen ist, dass das Konzentrat zum Behandlungsende t₃ aufgebraucht ist

Während der Kanister 12 vollständig entleert ist, verbleibt in dem Beutel 13 eine Restmenge an Konzentrat. Zum Entleeren des Beutels setzt die Steuer- und Recheneinheit 24 die Proportionierungspumpen 31 und 32 in Betrieb, so dass die Restmenge an Konzentrat aus dem Beutel 12 abgepumpt wird. Das Konzentrat wird in der Mischkammer 27 mit Wasser verdünnt, das die Pumpe 32 in einem vorgegebenen Volumenverhältnis zuführt Das verdünnte Konzentrat wird dann über die Abflussleitung 36 in den Abfluss 10 geleitet. Anschließend können sowohl der Kanister als auch der Beutel entnommen und korrekt entsorgt werden.

Für den Fall, dass während der eigentlichen Dialysebehandlung eine Komplikation auftritt, wird der Dialysator 1 abgetrennt und die Dialysierflüssigkeit über den Bypass 40 verworfen. Wenn häufig Störungen auftreten, kann es erforderlich sein, die Behandlung entsprechend zu verlängern, um die effektive Behandlungszeit T_{eff} zu erreichen. Bei dem obigen Ausführungsbeispiel ist dies allerdings nicht möglich, da nach Ablauf der Behandlungszeit zum Zeitpunkt t₃ kein Konzentrat mehr zur Verfügung steht.

In Fig. 2b ist der Blutfluß Qdₐ angegeben, den der Arzt bei einer konventionellen Dialysebehandlung einstellen würde, um eine bestimmte Dialysedosis vorzugeben. Deutlich ist zu erkennen, dass der Blutfluß mit dem erfindungsgemäßen Verfahren höher als der vom Arzt eingestellte Blutfluß Qdₐ ist, so daß die Dialysedosis erhöht wird.

Nachfolgend wird ein zweites Ausführungsbeispiel beschrieben, das es erlaubt, die Behandlung nach einer oder mehreren Unterbrechungen entsprechend zu verlängern. Das zweite Ausführungsbeispiel unterscheidet sich von dem ersten Ausführungsbeispiel nur dadurch, dass die Steuer- und Recheneinheit 24 der Versorgungsvorrichtung einen anderen Programmablauf vorgibt.

Zunächst wird wie bei dem ersten Ausführungsbeispiel ein Test durchgeführt. Nach Durchführung des Tests, bei dem Dialysierflüssigkeit verworfen wird, bestimmt die Steuer- und Recheneinheit 24 wieder die zu Beginn der Dialysebehandlung (zeitpunkt t₁) in dem Kanister 12 bzw. Beutel 13 befindliche Konzentratmenge und entscheidet welcher Behälter vollständig entleert werden soll. Es wird wieder angenommen, dass in dem Kanister 12 keine Restmenge an Konzentrat verbleiben soll.

Darauf hin gibt die Steuer- und Recheneinheit 24 für ein vorgegebenes Zeitintervall T_{B1}, das den wesentlichen Teil der Behandlung ausmachen sollte, eine Dialysierflüssigkeitsrate Qd vor, die gleich der in dem ersten Ausführungsbeispiel errechneten Rate ist. Die Rate kann grundsätzlich aber auch kleiner oder größer sein. Sie sollte aber derart bemessen sein, dass nach Ablauf des Zeitintervall T_{B1} zum Zeitpunkt t₂ noch eine ausreichende Konzentratmenge in dem Kanister 12 verbleibt, so dass die Behandlung noch über die vorgegebene Behandlungszeit T_{B} hinaus verlängert werden kann.

Nach Ablauf des Zeitintervalls T_{B1} stellt die Steuer- und Recheneinheit 37 der Dialysevorrichtung die Zeitdauer Tᵥ der Unterbrechung der Dialysebehandlung fest. Um diese Zeitdauer sollte die Behandlung über die vorgegebene Behandlungszeit T_{B} hinaus verlängert werden.

Die Steuer- und Recheneinheit 24 der Versorgungsvorrichtung berechnet nun aus der vorgegebenen Behandlungszeit T_{B}, dem vorgegebenen Zeitintervall T_{B1} und der Zeitdauer Tᵥ, um die die Behandlung verlängert werden soll, die noch verbleibende Behandlungszeit T_{B2},

Des weiteren berechnet die Steuer- und Recheneinheit 24 die in dem Kanister 12 zum Zeitpunkt t₂ befindliche Menge Mₜ₂ an Konzentrat aus der in der Eingabeeinheit 25 eingegebenen Konzentratmenge M₁, d.h. der vorgegebenen Konzentratmenge, und der Konzentratmenge, die während des Tests und dem vorgegebenen Zeitintervall T_{B1} verbraucht wurde.

Aus der verbleibenden Behandlungszeit T_{B2} und der in dem Kanister 12 befindlichen Konzentratmenge Mₜ₂ berechnet die Steuer- und Recheneinheit 24 dann die Dialysierflüssigkeitsrate Qdᵥ, die einzustellen ist, damit der Kanister 12 zum Behandlungsende vollständig entleert ist. Diese Dialysierflüssigkeitsrate wird dann für den Rest der Behandlung eingestellt.

Die Figuren 3a und 3b zeigen wieder die Konzentratmenge im Kanister 12 bzw. die Dialysierflüssigkeitsrate Qd als Funktion der Zeit t. Deutlich ist zu erkennen, dass die Behandlung über ein vorgegebenes Zeitintervall T_{B1} zunächst mit der gleichen Dialysierflüssigkeitsrate Qd_{b} wie bei dem ersten Ausführungsbeispiel vorgenommen wird, wobei nach Ablauf des Zeitintervalls T_{B1} eine niedrigere Dialysierflüssigkeitsrate Qdᵥ eingestellt wird, die derart bemessen ist, dass zum Ende der Behandlung der Kanister 12 vollständig entleert ist.

Deutlich ist wieder zu erkennen, dass der Blutfluß mit dem erfindungsgemäßen Verfahren höher als der Blutfluß Qdₐ ist, den der Arzt bei einer konventionellen Behandlung vorgeben würde.

Nach Ablauf der Behandlung wird die in dem Beutel 13 befindliche Restmenge an Konzentrat wieder mit Wasser verdünnt und verworfen, so dass sowohl der Kanister und als auch der Beutel entnommen und entsorgt werden können.

Zum Verwerfen der Restmenge an Konzentrat sind die Leitungen 26, 28, 29 und 36 sowie die zugehörigen Pumpen 30, 31 und 32 und die Mischkammer 27 grundsätzlich nicht erforderlich. Bei einer alternativen Ausführungsform schaltet die Steuer- und Recheneinheit 44 die Hämodialysevorrichtung zum Verwerfen der Restmenge an Konzentrat in den "Bypass", d.h. die Ventile 42 und 43 werden geschlossen und das Ventil 41 wird geöffnet, bis die Restmengen aufgebraucht sind. Diese Ausführungsform hat den Vorteil, dass eine zusätzliche Mischkammer sowie zusätzliche Leitungen und Pumpen nicht erforderlich sind. Daher dürfte diese Ausführungsform in der Praxis bevorzugt werden.

Es sei bemerkt, dass die erfindungsgemäße Versorgungsvorrichtung unterschiedlichste Vorgaben für die Dialysierflüssgkeitsrate erlaubt. Allein entscheidend ist, dass die Dialysierflüssigkeitsrate in Abhängigkeit von der Konzentratmenge, dem Volumenverhältnis und der Behandlungszeit eingestellt wird.

## Patentansprüche

1. Vorrichtung zur Versorgung eines Dialysators einer Dialysevorrichtung mit Dialysierflüssigkeit, mit
mindestens einer Aufnahmeeinheit (12, 13) für mindestens ein Dialysierflüssigkeitskonzentrat (K1, K2),
Mitteln (38) zum Bereitstellen von Wasser (W) zum Verdünnen des Dialysierflüssigkeitskonzentrats,
Mitteln (15; 17, 18, 20) zum Mischen von Dialysierflüssigkeitskonzentrat und Wasser in einem vorgegebenen Volumenverhältnis zur Herstellung der Dialysierflüssigkeit,
Mitteln (11) zum Zuführen der Dialysierflüssigkeit zu dem Dialysator der Dialysevorrichtung mit einer vorgegebenen Dialysierflüssigkeitsrate während einer vorgegebenen Behandlungszeit,
**dadurch gekennzeichnet, dass** eine Steuer- und Recheneinheit (24) vorgesehen ist, die derart ausgebildet ist, dass die Dialysierflüssigkeitsrate Qd in Abhängigkeit von einer vorgegebenen Menge an Dialysierflüssigkeitskonzentrat zu Beginn der Dialysebehandlung, von dem vorgegebenen Volumenverhältnis von Dialysierflüssigkeitskonzentrat und Wasser und von der vorgegebenen Behandlungszeit während der Dialysebehandlung derart eingestellt wird, dass zum Behandlungsende in der Aufnahmeeinheit (12, 13) eine vorgegebene Restmenge an Dialysierflüssigkeitskonzentrat oder keine Restmenge verbleibt.

2. Vorrichtung nach Anspruch 1, **dadurch gekennzeichnet, dass** die Steuer- und Recheneinheit (24) derart ausgebildet ist, dass aus der vorgegebenen Menge an Dialysierflüssigkeitskonzentrat zu Beginn der Dialysebehandlung, dem vorgegebenen Volumenverhältnis und der vorgegebenen Behandlungszeit die Dialysierflüssigkeitsrate Qd_{b} vor Beginn der Dialysebehandlung bestimmbar ist, die über die gesamte Behandlungszeit eingestellt wird, so dass zum Behandlungsende in der Aufnahmeeinheit (12, 13) die vorgegebene Restmenge an Dialysierflüssigkeitskonzentrat oder keine Restmenge verbleibt.

3. Vorrichtung nach Anspruch 1 oder 2, **dadurch gekennzeichnet, dass** die Steuer- und Recheneinheit (24) derart ausgebildet ist, dass für einen Test der Dialysevorrichtung vor Beginn der Dialysebehandlung in einem vorgegebenen Zeitintervall Tₜₑₛₜ aus der vorgegebenen Menge an Dialysierflüssigkeitskonzentrat zu Beginn der Behandlung und der während des vorgegebenen Zeitintervalls verbrauchten Menge an Dialysierflüssigkeitskonzentrat die in der Aufnahmeeinheit (12, 13) vor Beginn der Dialysebehandlung befindliche Menge an Dialysierflüssigkeitskonzentrat bestimmbar ist.

4. Vorrichtung nach einem der Ansprüche 1 bis 3, **dadurch gekennzeichnet, dass** die Steuer- und Recheneinheit (24) mit den Mitteln (15; 17, 18, 20) zum Mischen von Dialysierflüssigkeitskonzentrat und Wasser derart zusammenwirkt, dass in einem vorgegebenen Zeitintervall T_{B1} der Dialysebehandlung eine vorgegebene Dialysierflüssigkeitsrate Qd_{b} einstellbar ist, wobei während der Dialysebehandlung aus der vorgegebenen Menge an Dialysierflüssigkeitskonzentrat zu Beginn der Dialysebehandlung und der verbrauchten Menge an Dialysierflüssigkeitskonzentrat, die nach Ablauf des Zeitintervalls in der Aufnahmeeinheit (12, 13) befindliche Menge an Dialysierflüssigkeitskonzentrat bestimmbar ist, und dass nach Ablauf des vorgegebenen Zeitintervalls T_{B1} aus der in der Aufnahmeeinheit befindlichen Menge an Dialysierflüssigkeitskonzentrat, dem vorgegebenen Volumenverhältnis und der verbleibenden Behandlungszeit die Dialysierflüssigkeitsrate Qdᵥ bestimmbar ist, die über die verbleibende Behandlungszeit eingestellt wird, so dass zum Behandlungsende in der Aufnahmeeinheit die vorgegebene Restmenge an Dialysierflüssigkeitskonzentrat oder keine Restmenge verbleibt.

5. Vorrichtung nach einem der Ansprüche 1 bis 4, **dadurch gekennzeichnet, dass** Mittel (26, 28; 30, 31) zum Abführen der Restmenge an Dialysierflüssigkeit aus der Aufnahmeeinheit (12, 13) in einen Ablauf (10) vorgesehen sind, wobei die Steuer- und Recheneinheit (24) derart mit den Mitteln zum Abführen der Restmenge an Dialysierflüssigkeit zusammenwirkt, dass die vorgegebene Restmenge nach Ablauf der Dialysebehandlung in den Ablauf abführbar ist.

6. Vorrichtung nach einem der Ansprüche 1 bis 5, **dadurch gekennzeichnet, dass** Mittel (27) zum Mischen der Restmenge an Dialysierflüssigkeit mit Wasser in einem vorgegebenen Volumenverhältnis vorgesehen sind, wobei die Steuer- und Recheneinheit (24) derart mit den Mitteln zum Mischen der Restmenge mit Wasser zusammenwirkt, dass die Restmenge der Dialysierflüssigkeit in einem vorgegebenen Volumenverhältnis mit Wasser verdünnbar ist, bevor die Restmenge in den Ablauf (10) abgeführt wird.

7. Vorrichtung nach einem der Ansprüche 1 bis 4, **dadurch gekennzeichnet, dass** in der Aufnahmeeinheit (12, 13) keine Restmenge an Dialysierflüssigkeit verbleibt.

8. Vorrichtung nach einem der Ansprüche 1 bis 7, **dadurch gekennzeichnet, dass** Mittel (25) zum Eingeben der in der Aufnahmeeinheit (12, 13) vorgegebenen Menge an Dialysierflüssigkeitskonzentrat, des vorgegebenen Volumenverhältnisses von Dialysierflüssigkeitskonzentrat und Wasser und der vorgegebenen Behandlungszeit vorgesehen sind.

9. Vorrichtung nach einem der Ansprüche 1 bis 7, **dadurch gekennzeichnet, dass** die Daten, die die vorgegebene Menge an Dialysierflüssigkeitskonzentrat und/oder das vorgegebene Volumenverhältnis von Dialysierflüssigkeitskonzentrat und Wasser und/oder die vorgegebene Behandlungszeit beschreiben, in Form eines Strichcodes auf der Aufnahmeeinheit (12, 13) hinterlegt sind.

## Claims

1. A device for supplying a dialyser of a dialysis unit with dialysis fluid, comprising
at least one receiving unit (12, 13) for at least one dialysis fluid concentrate (K1, K2),
means (38) for providing water (W) for diluting the dialysis fluid concentrate,
means (15; 17, 18, 20) for mixing the dialysis fluid concentrate and the water in a predetermined volume ratio to prepare the dialysis fluid,
means (11) for feeding the dialysis fluid to the dialyser of the dialysis unit with a predetermined dialysis fluid flow rate during a predetermined treatment time,
**characterized in that** a control and calculating unit (24) is provided that is designed such that the dialysis fluid flow rate Qd is set during the dialysis treatment in dependence on a predetermined amount of dialysis fluid concentrate at the beginning of the dialysis treatment, on the predetermined volume ratio of dialysis fluid concentrate and water and on the predetermined treatment time in such a manner that at the end of the treatment, a predetermined residual amount of dialysis fluid concentrate or no residual amount remains in the receiving unit (12, 13).

2. The device according to claim 1, **characterized in that** the control and calculating unit (24) is designed such that from the predetermined amount of dialysis fluid concentrate at the beginning of the dialysis treatment, the predetermined volume ratio and the predetermined treatment time, the dialysis fluid flow rate Qd_{b} can be determined prior to the beginning of the dialysis treatment, which is set over the entire treatment time so that at the end of the treatment, the predetermined residual amount of dialysis fluid concentrate or no residual amount remains in the receiving unit (12, 13).

3. The device according to claim 1 or claim 2, **characterized in that** the control and calculating unit (24) is designed such that for a test of the dialysis unit prior to the beginning of the dialysis treatment and within a predetermined time interval Tₜₑₛₜ, the amount of dialysis fluid concentrate contained in the receiving unit (12, 13) prior to the beginning of the dialysis treatment can be determined from the predetermined amount of dialysis fluid concentrate at the beginning of the treatment and the amount of dialysis fluid concentrate used up during the predetermined time interval.

4. The device according to any one of the claims 1 to 3, **characterized in that** the control and calculating unit (24) interacts with the means (15; 17, 18, 20) for mixing dialysis fluid concentrate and water in such a manner that within a predetermined time interval T_{B1} of the dialysis treatment, a predetermined dialysis fluid flow rate Qd_{b} can be set, wherein during the dialysis treatment, the amount of dialysis fluid concentrate contained in the receiving unit (12, 13) at the end of the time interval can be determined from the predetermined amount of dialysis fluid concentrate at the beginning of the dialysis treatment and the amount of dialysis fluid used up, and that at the end of the predetermined time interval T_{B1}, from the amount of dialysis fluid concentrate in the receiving unit, the predetermined volume ratio and the remaining treatment time, the dialysis fluid flow rate Qdᵥ can be determined, which is set over the remaining treatment time so that at the end of the treatment, the predetermined residual amount of dialysis fluid concentrate or no residual amount remains in the receiving unit.

5. The device according to any one of the claims 1 to 4, **characterized in that** means (26, 28; 30, 31) are provided for discharging the residual amount of dialysis fluid from the receiving unit (12, 13) into a drain (10), wherein the control and calculating unit (24) interacts with the means for discharging the residual amount of dialysis fluid in such a manner that at the end the dialysis treatment, the predetermined residual amount can be discharged into the drain.

6. The device according to any one of the claims 1 to 5, **characterized in that** means (27) are provided for mixing the residual amount of dialysis fluid with water in a predetermined volume ratio, wherein the control and calculating unit (24) interacts with the means for mixing the residual amount with water in such a manner that the residual amount of the dialysis fluid can be diluted with water in a predetermined volume ratio before the residual amount is discharged into the drain (10).

7. The device according to any one of the claims 1 to 4, **characterized in that** no residual amount of dialysis fluid remains in the receiving unit (12, 13).

8. The device according to any one of the claims 1 to 7, **characterized in that** means (25) are provided for entering the predetermined amount of dialysis fluid concentrate in the receiving unit (12, 13), the predetermined volume ratio of dialysis fluid concentrate and water, and the predetermined treatment time.

9. The device according to any one of the claims 1 to 7, **characterized in that** the data describing the predetermined amount of dialysis fluid concentrate and/or the predetermined volume ratio of dialysis fluid concentrate and water and/or the predetermined treatment time are deposited in the form of a barcode on the receiving unit (12, 13).

## Revendications

1. Dispositif pour l'alimentation d'un dialyseur d'un appareil de dialyse en liquide de dialyse, comprenant
au moins une unité réceptrice (12, 13) dédiée à au moins un concentré de liquide de dialyse (K1, K2),
des moyens (38) de fourniture en eau (W), en vue de la dilution dudit concentré de liquide de dialyse,
des moyens (15 ; 17, 18, 20) de mélange d'eau et de concentré de liquide de dialyse, selon un rapport de volume préétabli, en vue de produire le liquide de dialyse,
des moyens (11) de délivrance dudit liquide de dialyse au dialyseur de l'appareil de dialyse, selon un débit de liquide de dialyse préétabli durant un temps de traitement préétabli,
**caractérisé par le fait qu'**il est prévu une unité (24) de commande et de calcul, réalisée de manière que le débit Qd de liquide de dialyse soit réglé en fonction d'une quantité préétablie de concentré de liquide de dialyse au début du traitement dialytique, du rapport de volume préétabli d'eau et de concentré de liquide de dialyse, et du temps de traitement préétabli au cours dudit traitement dialytique, de telle sorte qu'il subsiste dans l'unité réceptrice (12, 13), à la fin du traitement, une quantité résiduelle préétablie de concentré de liquide de dialyse, ou une quantité résiduelle nulle.

2. Dispositif selon la revendication 1, **caractérisé par le fait que** l'unité (24) de commande et de calcul est réalisée de manière que le débit Qd_{b} de liquide de dialyse réglé pour la totalité du temps de traitement puisse être estimé, avant le début du traitement dialytique, sur la base de la quantité préétablie de concentré de liquide de dialyse au début dudit traitement dialytique, du rapport de volume préétabli et du temps de traitement préétabli, de telle sorte qu'il subsiste dans l'unité réceptrice (12, 13), à la fin du traitement, la quantité résiduelle préétablie de concentré de liquide de dialyse, ou une quantité résiduelle nulle.

3. Dispositif selon la revendication 1 ou 2, **caractérisé par le fait que** l'unité (24) de commande et de calcul est réalisée de manière que la quantité de concentré de liquide de dialyse, située dans l'unité réceptrice (12, 13) avant le début du traitement dialytique, puisse être estimée pour un test de l'appareil de dialyse avant le début dudit traitement dialytique, dans un intervalle de temps préétabli Tₜₑₛₜ, sur la base de la quantité préétablie de concentré de liquide de dialyse au début dudit traitement, et de la quantité de concentré de liquide de dialyse consommée au cours dudit intervalle de temps préétabli.

4. Dispositif selon l'une des revendications 1 à 3, **caractérisé par le fait que** l'unité (24) de commande et de calcul coopère avec les moyens (15 ; 17, 18, 20) de mélange d'eau et de concentré de liquide de dialyse, de façon telle qu'un débit préétabli Qd_{b} de liquide de dialyse puisse être réglé dans un intervalle de temps préétabli T_{B1} du traitement dialytique, sachant que la quantité de concentré de liquide de dialyse située dans l'unité réceptrice (12, 13) après expiration de l'intervalle de temps peut être déterminée, au cours du traitement dialytique, sur la base de la quantité préétablie de concentré de liquide de dialyse au début dudit traitement dialytique, et de la quantité de concentré de liquide de dialyse consommée, et qu'il est possible d'estimer après expiration dudit intervalle de temps préétabli T_{B1}, sur la base de ladite quantité de concentré de liquide de dialyse située dans l'unité réceptrice, du rapport de volume préétabli et du temps de traitement restant, le débit Qdᵥ de liquide de dialyse réglé pour ledit temps de traitement restant, de telle sorte qu'il subsiste dans ladite unité réceptrice, à la fin du traitement, la quantité résiduelle préétablie de concentré de liquide de dialyse, ou une quantité résiduelle nulle.

5. Dispositif selon l'une des revendications 1 à 4, **caractérisé par le fait que** des moyens (26, 28 ; 30, 31) sont prévus pour évacuer la quantité résiduelle de liquide de dialyse vers un écoulement (10), à partir de l'unité réceptrice (12, 13), l'unité (24) de commande et de calcul coopérant avec lesdits moyens d'évacuation de la quantité résiduelle de liquide de dialyse, de façon telle que la quantité résiduelle préétablie puisse être évacuée vers ledit écoulement après achèvement du traitement dialytique.

6. Dispositif selon l'une des revendications 1 à 5, **caractérisé par le fait que** des moyens (27) sont prévus pour mélanger la quantité résiduelle de liquide de dialyse avec de l'eau, selon un rapport de volume préétabli, l'unité (24) de commande et de calcul coopérant avec lesdits moyens de mélange de ladite quantité résiduelle avec de l'eau, de façon telle que la quantité résiduelle du liquide de dialyse puisse être diluée par de l'eau, selon un rapport de volume préétabli, avant que ladite quantité résiduelle soit évacuée vers l'écoulement (10).

7. Dispositif selon l'une des revendications 1 à 4, **caractérisé par le fait qu'**aucune quantité résiduelle de liquide de dialyse ne subsiste dans l'unité réceptrice (12, 13).

8. Dispositif selon l'une des revendications 1 à 7, **caractérisé par le fait que** des moyens (25) sont prévus pour entrer la quantité de concentré de liquide de dialyse préétablie dans l'unité réceptrice (12, 13), le rapport de volume préétabli d'eau et de concentré de liquide de dialyse, et le temps de traitement préétabli.

9. Dispositif selon l'une des revendications 1 à 7, **caractérisé par le fait que** les données, descriptives de la quantité préétablie de concentré de liquide de dialyse, et/ou du rapport de volume préétabli d'eau et de concentré de liquide de dialyse, et/ou du temps de traitement préétabli, sont consignées sur l'unité réceptrice (12, 13) sous la forme d'un code-barres.
